# EUROPEAN PATENT APPLICATION

(11) **EP 2 835 422 A1**
(43) Date of publication of application: **11.02.2015**
(21) Application number: 13003933.2
(22) Date of filing: 06.08.2013
(51) Int. Cl.: C12N 9/96

(54) **Method for the isolation of a biologically active target compound**

(71) Applicant: Technische Universität München, 80333 München (DE)
(72) Inventor: Parlar, Harun, 85406 Zolling (DE)
(74) Representative: Hartz, Nikolai

(57) **Abstract**

A method for the isolation of a biologically active target compound from an aqueous mixture, said method comprising the following steps:
(a) providing a first aqueous mixture containing the target compound;
(b) selectively and reversibly reacting a surface-active catcher compound with the target compound in the aqueous mixture at a first predetermined pH for forming a reversibly bound complex between the catcher compound and the target compound;
(c) foaming the liquid mixture for providing a foam fraction containing the reversibly bound complex from the first aqueous mixture;
(d) transferring the foam fraction into a second aqueous mixture;
(e) precipitating the catcher compound while the target compound remains dissolved in the second aqueous mixture by adjusting the pH of the second aqueous mixture and/or adjusting the ion strength of the second aqueous mixture; and
(f) isolating the target compound from the second aqueous mixture as a biologically active target compound.

## Description

### Field of the Invention

The present invention relates to a method for the isolation of a biologically active target compound from an aqueous mixture.

Moreover, the present invention relates to a reversibly bound complex between a catcher compound and the target compound, which complex is an intermediate of the method of the present invention.

Furthermore, the present invention relates to a use of the reversibly bound complex between a catcher compound and the target compound for providing a storage-stable form of the target compound.

According to the present invention, it is possible to selectively isolate a biologically active target compound even when present in low concentration from an aqueous mixture by formation of a specific reversibly bound complex between a catcher compound and the target compound. The reversibly bound complex may is adapted to forming a foam and may be treated according to the present invention in order to release the target compound in a biologically active form.

### Background of the Invention

Adsorptive bubble separation is a process in which a material is concentrated or separated by selectively adsorbing the material to the surface of gas bubbles rising through a liquid, cf. Adsorptive Bubble Separation Techniques, Robert Lemlich, Editor, Academic Press, New York, NY, 1972.

Tweezing-adsorptive bubble separation is known from Gerken, M. et al. Anal. Chem. 2005 Oct 1; 77(19):6113-7 for the enrichment of metalloenzymes based on the chelation of the enzymes' active center with, for example, N-octylcarbamoylmethyliminodiacetic acid (ADA-C8). Tweezing-ABS has been shown to selectively and effectively enrich metalloenzymes.

However, the products obtainable according to the process disclosed in the prior art are unsatisfactory with regard to purity due to residual amounts of catcher compounds used in the tweezing bubble separation. In order to provide high purity products, it is necessary to provide the biologically active target compound with higher purity.

### Summary of the Invention

It is the problem of the present invention to provide a method for the isolation of a biologically active target compound from an aqueous mixture whereby the target compound may be selectively recovered in a biologically active form, and which allows the simple and efficient elimination of any auxiliary agent.

Accordingly, the present invention provides a method for the isolation of a biologically active target compound from an aqueous mixture, said method comprising the following steps:
(a) providing a first aqueous mixture containing the target compound;
(b) selectively and reversibly reacting a surface-active catcher compound with the target compound in the aqueous mixture at a first predetermined pH for forming a reversibly bound complex between the catcher compound and the target compound;
(c) foaming the liquid mixture for providing a foam fraction containing the reversibly bound complex from the first aqueous mixture;
(d) transferring the foam fraction into a second aqueous mixture;
(e) precipitating the catcher compound while the target compound remains dissolved in the second aqueous mixture by adjusting the pH of the second aqueous mixture and/or adjusting the ion strength of the second aqueous mixture; and
(f) isolating the target compound from the second aqueous mixture as a biologically active target compound.

In case the biologically active target compound is a metalloenzyme, the method preferably further comprises adding a metal compound to the second aqueous mixture for separating the catcher compound from the target compound. The metal compound is added preferably after transferring of the foam fraction into a second aqueous mixture and after the catcher compound was precipitated by adjusting the pH of the second aqueous mixture and/or adjusting the ion strength of the second aqueous mixture. Accordingly, traces of the catcher compound may be eliminated from the mixture by precipitation of the catcher compound as a complex or salt with the metal ion of the metal compound while the biologically active compound remains in solution and may be stabilized by the excess of metal ions present in the solution.

Moreover, it is the problem of the present invention to provide a reversibly bound complex between a catcher compound and the target compound, which is obtainable by drying the foam obtained in step (c) of the method according to the invention.

Accordingly, the present invention provides a reversibly bound complex between a catcher compound and the target compound, which is obtainable by drying the foam obtained in step (c) of the method according to the invention.

Finally, the present invention provides the use of the reversibly bound complex between a catcher compound and the target compound according to the present invention for providing a storage stable form of the target compound.

The present invention starts out from the recognition that it is possible to selectively forming a surface active reversibly bound complex between a specific catcher compound and a biologically active target compound, which complex may be isolated as a foam. Moreover, the present invention is based on the recognition that the efficiency of the isolation of the target compound from the reversibly bound complex in a biologically active form may be increased while at the same time the target compound may be stabilized in case a specific additive and/or pH is used in order to precipitate the catcher compound, and preferably in case a metal compound is added to the second aqueous mixture for separating the catcher compound from the target compound as a precipitate.

### Detailed Description of Preferred Embodiments

The method of the present invention allows the isolation of a biologically active target compound from an aqueous mixture.

The biologically active target compound may be selected from a metalloenzyme, an enzyme, a metalloprotein, an immunoglobulin, a small molecule, or a metal compound.

According to a preferred embodiment, the biologically active target compound is a metalloprotein, in particular a metalloenzyme selected from oxydases and reductases, in particular laccases or peroxidases.

An oxidase is an enzyme that catalyzes an oxidation-reduction reaction involving molecular oxygen (O₂) as the electron acceptor whereby oxygen is reduced to water (H₂O) or hydrogen peroxide (H₂O₂). Specific examples of oxidases are laccase, glucose oxidase, monoamine oxidase, cytochrome P450 oxidase, NADPH oxidase, xanthine oxidase, L-gulonolactone oxidase, and lysyl oxidase.

Laccases are copper-containing oxidase enzymes that are found in many plants, fungi, and microorganisms. The copper is bound in several sites; Type 1, Type 2, and/or Type 3. The ensemble of types 2 and 3 copper is called a trinuclear cluster. Type 1 copper is available to action of solvents, such as water. Laccases act on phenols and similar molecules, performing a one-electron oxidation.

According to a further preferred embodiment, the enzymes may be selected from pyrophosphatases.

The aqueous mixture may be obtained from a biological source including a fluid of an animal or plant, such as a body fluid or a culture broth of a microorganism.

The method according to the present invention comprises a step of providing a first aqueous mixture containing the target compound. The target compound may be present in the aqueous mixture in a concentration of from 0.01 mg/l (w/v) to 10 g/l (w/v), preferably in a concentration of from 0.1 mg/l (w/v) to 1 g/l (w/v).

The first aqueous mixture may contain only the biologically active target compound or the first aqueous mixture may contain additional compounds besides the biologically active target compound. The additional compounds may be selected from salts, polymeric materials, particulate material, water-miscible liquids and organic small molecules. The total of any additional compounds in the aqueous mixture may be present in a concentration of from 0.01 mg/l (w/v) to 20 g/I (w/v).

Moreover, the method of the present invention comprises the step of selectively and reversibly reacting a surface-active catcher compound with the target compound in the aqueous mixture at a first predetermined pH for forming a reversibly bound complex between the catcher compound and the target compound.

The reaction condition for the reaction of the surface-active catcher compound with the target compound in the first aqueous mixture are not particularly limited as long as the formation of the formation of a reversibly bound complex between the catcher compound and the biologically active target compound is not impaired. Specifically, the reaction temperature may be in the range of between 0 °C to 95 °C, more preferably between 10 °C and 50 °C. The reaction time is not particularly limited and is usually in the range of from 3 minutes to 3 180 minutes, more preferably 15 minutes to 60 minutes.

The pH of the aqueous mixture is preferably adjusted in order to provide the highest possible stability of the complex. Preferably, the pH of the aqueous mixture is adjusted in the range of from 5 to 7.5.

According to a preferred embodiment, the surface active catcher compound comprises a moiety selectively and reversibly bonding to the target compound and a straight chain or branched C₄₋₁₄ hydrocarbon group having 1 to 3 nitrogen atoms as primary, secondary or tertiary amine groups, preferably wherein the catcher compound comprises a C₄₋₁₄ alkyl group containing 1 to 3 primary, secondary or tertiary amine groups.

Preferably, the catcher is a molecule of the following formula (I): 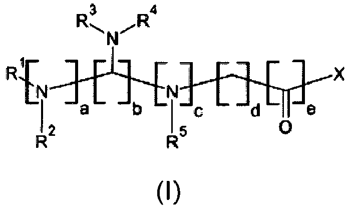 wherein
X is a moiety selectively and reversibly bonding to the target compound,
R¹ and R² which are independent from each other, represent a hydrogen atom or a straight chain or branched C₁₋₁₄ alkyl group;
R³ and R⁴ which are independent from each other, represent a hydrogen atom or a straight chain or branched C₁₋₁₄ alkyl group;
R⁵ represents a hydrogen atom or a straight chain or branched C₁₋₁₄ alkyl group;
a is an integer of 0 or 1,
b is an integer of 0, 1 or 2,
c is an integer of 0 or 1,
d is an integer of 0 to 14, and
e is an integer of 0 or 1,
provided that the total number of carbon atoms of the molecule other than X is from 4 to 14.

In case a is 0, then R² is absent. In case a is 1, R² is present. If R² is present, one of R¹ and R² preferably represents a hydrogen atom and the other of R¹ and R² represents a straight chain or branched C₁₋₁₄ alkyl group, more preferably C₄₋₁₀ alkyl group.

In case b is 0, R³ and R⁴ are absent. In case b is 1, R³ and R⁴ are present. If R³ and R⁴ are present, one of R³ and R⁴ preferably represents a hydrogen atom and the other of R³ and R⁴ represents a straight chain or branched C₁₋₁₄ alkyl group, more preferably C₄₋₁₀ alkyl group.

In case c is 0, then R⁵ is absent. In case a is 1, R⁵ is present. If R⁵ is present, R⁵ preferably represents a hydrogen atom.

According to a specific embodiment, d is 0. According to a further specific embodiment, a, b, and d are each 0.

Preferably, X is a moiety of the following formula (II) 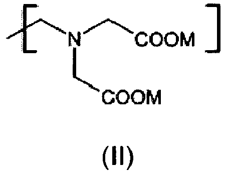 wherein M is a hydrogen atom.

According to a preferred embodiment, the catcher compound has at least one straight or branched alkyl group containing 4 to 14 carbon atoms as R¹, R², R³, R⁴ or R⁵. According to a particularly preferred embodiment, the surface-active catcher compound is N-octyl-carbamoylmethyl iminodiacetic acid (ADA-C8).

Advantageously, the HLB value of the catcher compound is in the range of from 15 to 20. The hydrophilic-lipophilic balance (HLB) of a surfactant is a measure of the degree to which it is hydrophilic or lipophilic, determined by calculating values for the different regions of the molecule. Accordingly, the catcher compound is a hydrotope.

Furthermore, the method of the present invention comprises the step of foaming the liquid mixture for providing a foam fraction containing the reversibly bound complex from the first aqueous mixture. The conditions of the foaming step are not particularly limited provided that the formation of a foam is not impaired. Accordingly, it is possible to use mechanical stirring of the aqueous mixture or the introduction of a gas into the aqueous mixture in order to provide a foam. The foaming time is not particularly limited and may be selected from the range of from 3 minutes to 24 hours, more preferably from 10 minutes to 3 hours.

Furthermore, the method of the present invention comprises a step of transferring the foam fraction into a second aqueous mixture.

The method of the present invention further comprises precipitating the catcher compound while the target compound remains dissolved in the second aqueous mixture by adjusting the pH of the second aqueous mixture and/or adjusting the ion strength of the second aqueous mixture.

According to a preferred embodiment, the biologically active target compound is a metalloprotein, in particular a metalloenzyme, and the method further comprises adding a metal compound to the second aqueous mixture for separating the catcher compound from the target compound. A metalloprotein may be any protein that contains a metal ion cofactor. In case of a metalloenzymes, metal ions are bound to the protein with one labile coordination site. In view of the biological activity of the metalloenzymes, the presence of the metal ions and the shape of the active site is crucial. Accordingly, it is preferred to stabilize the active site of the metalloenzyme and at the same time precipitate the catcher compound by the addition of a suitable metal compound. Preferably, the metal of the metal ion cofactor and the metal of the metal compound added according to the present invention are the same elements.

Accordingly, in case the metal compound is a magnesium compound, the metalloenzyme is selected from glucose 6-phosphatase, hexokinase, and DNA polymerase. In case the metal compound is a vanadium compound, the metalloenzyme is preferably selected from vanabins. In case the metal compound is a manganese compound, the metalloenzyme is preferably selected from arginase. In case the metal compound is an iron compound, the metalloenzyme is preferably selected from catalase, hydrogenase, IRE-BP, and aconitase. In case the metal compound is a nickel compound, the metalloenzyme is preferably selected from urease and hydrogenase. In case the metal compound is a copper compound, the metalloenzyme is preferably selected from cytochrome oxidase and laccase. In case the metal compound is a zinc compound, the metalloenzyme is preferably selected from alcohol dehydrogenase, carboxypeptidase, aminopeptidase, and beta amyloid. In case the metal compound is a molybdenum compound, the metalloenzyme is preferably selected from nitrate reductases. In case the metal compound is a selenium compound, the metalloenzyme is preferably selected from glutathione peroxidases.

Preferably, the metal compound is a water soluble salt of a metal ion selected from Mg²⁺, Cu²⁺, Fe²⁺, Fe³⁺, Co²⁺, Zn²⁺, V²⁺ or Ni²⁺.

Specifically, in case the biologically active compound is laccase it is preferred to add metal ions present in the catalytic center of the laccase, in order to stabilize the laccase and in order to precipitate the catcher compound. Specifically, it is preferred to add an aqueous solution of a Cu²⁺-salt such as CuSO₄•5H₂O for precipitating the catcher compound while the target compound remains dissolved in the second aqueous mixture.

Moreover, it is preferred to lower the pH of the second aqueous mixture for precipitating the catcher compound while the target compound remains dissolved in the second aqueous mixture. According to a preferred embodiment, the first predetermined pH is in the range of from 4 to 7.5 and the difference between the first predetermined pH and the second predetermined pH is at least 1, whereby the second pH is in the range of from 3 to 6.5.

The amount of the metal compound is not particularly limited and may be selected in the range of from 0.01 mg/l (w/v) to 20 g/l (w/v), more preferably 1.0 mg/l (w/v) to 1.0 g/l (w/v),

The pH may be lowered by the addition of an acid. The acid may be selected from inorganic acids such as hydrochloric acid, sulphuric acid and phosphoric acid, or organic acids such as acetic acid.

Accordingly, it is possible to eliminate the catcher compound from the from the second aqueous mixture by filtration.

Finally, the method of the present invention comprises isolating the target compound from the second aqueous mixture as a biologically active target compound. The isolation of the biologically active compound may be carried our by any suitable method including lyophilization, chromatography or distillation.

The precipitation of the catcher compound is preferably due to the adjustment of the pH to the second predetermined pH. Additionally, the precipitation of the catcher compound is due to the addition of a precipitating compound.

According to a preferred embodiment, the biologically active target compound is an metalloenzyme, and the method further comprises adding a metal compound to the second aqueous mixture for separating the catcher compound from the target compound.

The present invention also relates to a reversibly bound complex between a catcher compound and the target compound, which is obtainable by drying the foam obtained in step (c) of the method of the present invention. The drying step may be carried out under reduced pressure.

The reversibly bound complex between a catcher compound and the target compound may be used for providing a storage stable form of the target compound.

The present invention will now be further illustrated based on the following specific examples.

### EXPERIMENTAL SECTION

**Synthesis of N-Octylcarbamoylmethyliminodiacetic Acid.** ADA-C8 was synthesized by solving iminodiacetic acid (1.33 g, 10 mmol) and N-octyl-2-chloroacetamide (2.06 g, 10 mmol) in 50 mL of ethanol. Sodium hydroxide (10%) was used to adjust the solution to pH 11. The mixture was refluxed for 5 h and kept constantly at pH 11 by adding sodium hydroxide (10%). Thereafter, the solution was evaporated to dryness. The residue was diluted with 80 mL of distilled water and extracted with diethyl ether. Concentrated hydrochloric acid (37%) was added to the aqueous phase until pH 2. The obtained precipitate was filtered and dried under vacuum. A yield of 71% (2.16 g or 7.13 mmol) was obtained. The substance was characterized by ¹H NMR and ¹³C NMR spectroscopy: (Bruker, AC 250) ¹H NMR (250 MHz, D₆-DMSO) δ 0.85 (t, ³J = 6.5 Hz, 3H, CH₃), 1.24 (br m, 10H, CH₂), 1.39 (m, 2H, CH₂), 3.08 (m, 2H, NHCH₂CH₂), 3.28 (s, 2H, NCH₂CO), 3.43 (s, 4H, CH₂COOH), 8.02 (t, ³J = 5.8 Hz, 1H, NH). ¹³C NMR (62.5 MHz, D₆-DMSO) δ 13.9 (CH₃), 22.1 (CH₂), 26.3 (CH₂), 28.6 (CH₂), 28.7 (CH₂), 29.1 (CH₂), 31.2 (CH₂), 38.2 (CH₂-N), 55.4 (2C, CH₂-COOH), 58.0 (N-CH₂COOH), 170.4 (CONH), 172.7 (2C, COOH).

**Preparation of the Enzymatic Solution**. The enzymatic solution (starting solution) consists of laccase C (EC 1.10.3.2, from *Trametes sp.,* obtained from ASA "Spezialenzyme", Wolfenbuettel, Germany; 11.4 units L⁻¹) in a fermentation broth prepared by dissolving 30 g of glucose monohydrate, 4.5 g of L-asparagine monohydrate, 3.0 g of yeast extract, 1.5 g of KH₂PO₄, 0.5 g L⁻¹ MgSO₄, and 1.0 mL of mineral solution in 1 L demineralized water. All other chemicals were obtained from Fluka.

**Measurement of the Enzymatic Activity**. The enzymatic activity is measured by a photometric assay at pH 4.5, 6.0, or 7.0 depending on chelation conditions using 3-ethylbenzthiazoline- 6-sulfonic acid as substrate. Therefore, 100 µL of the laccase C solution, 800 µL of buffer, and 100 µL of substrate were mixed in a semimicrocuvette.

**Chelation.** For the chelation of laccase C, between 5 and 100 mg mL⁻¹ N-octylcarbamoylmethyliminodiacetic acid (ADA-C8) is added to the enzymatic broth at a pH of 4.5, 6.0, or 7.0.

**Adsorptive Bubble Separation Experiments**. A starting solution is mixed each withs ADA-C8. An amount of 2.75 mL is placed in a cylindrical glass column (0.15- and 11-mm i.d.). Foam emerges at room temperature after compressed air is introduced through a glass frit (porosity 16-40 µm). The enrichment (ER) and recovery (R) may be influenced by optimizing conditions of gas flow rate, pH of the starting solution, initial concentration of the surfactant, and foaming period.

**Separation of the foam and dechelation by lowering pHand addition of metal compound.** The foam is separated from the column and transferred into an aqueous mixture. For dechelation of ADA-C8, the pH of the enzymatic broth is lowered to pH 3.5 by the addition of HCl and an excess of CuSO₄•5H₂O is added. Additional HCl may be added until pH 3, which causes precipitation. Thus, the chelator may be eliminated from the enzymatic solution by filtration.

With the help of the catcher (β-ADA-N-C8) and CuSO₄•5H₂O, laccase C could be isolated with a yield of 99.9 % without loss of enzyme activity.

## Claims

1. A method for the isolation of a biologically active target compound from an aqueous mixture, said method comprising the following steps:
(a) providing a first aqueous mixture containing the target compound;
(b) selectively and reversibly reacting a surface-active catcher compound with the target compound in the aqueous mixture at a first predetermined pH for forming a reversibly bound complex between the catcher compound and the target compound;
(c) foaming the liquid mixture for providing a foam fraction containing the reversibly bound complex from the first aqueous mixture;
(d) transferring the foam fraction into a second aqueous mixture;
(e) precipitating the catcher compound while the target compound remains dissolved in the second aqueous mixture by adjusting the pH of the second aqueous mixture and/or adjusting the ion strength of the second aqueous mixture; and
(f) isolating the target compound from the second aqueous mixture as a biologically active target compound.

2. The method according to claim 1, wherein the biologically active target compound is an metalloenzyme, and the method further comprises adding a metal compound to the second aqueous mixture for separating the catcher compound from the target compound.

3. The method according to claim 1, wherein the surface active catcher compound comprises a moiety selectively and reversibly bonding to the target compound and a straight chain or branched C₄₋₁₄ hydrocarbon group having 1 to 3 nitrogen atoms as primary, secondary or tertiary amine groups, preferably wherein the catcher compound comprises a C₄₋₁₄ alkyl group containing 1 to 3 primary, secondary or tertiary amine groups.

4. The method according to any one of the preceding claims, wherein the catcher is a molecule of the following formula (I): 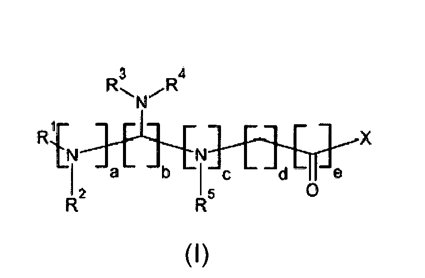 wherein
X is a moiety selectively and reversibly bonding to the target compound,
R¹ and R² which are independent from each other, represent a hydrogen atom or a straight chain or branched C₁₋₁₄ alkyl group;
R³ and R⁴ which are independent from each other, represent a hydrogen atom or a straight chain or branched C₁₋₁₄ alkyl group;
R⁵ represents a hydrogen atom or a straight chain or branched C₁₋₁₄ alkyl group;
a is an integer of 0 or 1,
b is an integer of 0, 1 or 2,
c is an integer of 0 or 1,
d is an integer of 0 to 14, and
e is an integer of 0 or 1,
provided that the total number of carbon atoms of the molecule other than X is from 4 to 14.

5. The method according to claim 4, wherein X is a moiety of the following formula (II) 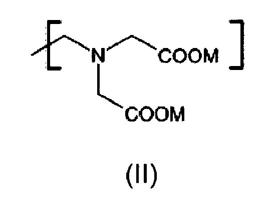 wherein M is a hydrogen atom.

6. The method according to claim 5, wherein the surface-active catcher compound is N-octyl-carbamoylmethyl iminodiacetic acid (ADA-C8).

7. The method according to any one of the preceding claims, wherein the HLB value of the catcher compound is in the range of from 15 to 20.

8. The method according to any one of the preceding claims, wherein first predetermined pH is in the range of from 5 to 7.5 and the difference between the first predetermined pH and the second predetermined pH is at least 1, whereby the second pH is in the range of from 3 to 10.

9. The method according to any one of the preceding claims, wherein the precipitation of the catcher compound is due to the adjustment of the pH to the second predetermined pH.

10. The method according to any one of claims 1 to 8, wherein the precipitation of the catcher compound is due to the addition of a precipitating compound.

11. The method acceding to any one of the preceding claims wherein the biologically active target compound is selected from a metalloenzyme, an enzyme, a metalloprotein, an immunoglobulin, a small molecule, or a metal compound.

12. The method according to claim 11, wherein the metalloenzyme is selected from oxydases, reductases, laccases, or peroxidases.

13. The method according to claim 11, wherein the enzymes are selected from pyrophosphatases.

14. A reversibly bound complex between a catcher compound and the target compound, which is obtainable by drying the foam obtained in step (c) of the method of any one of claims 1 to 13.

15. Use of the reversibly bound complex between a catcher compound and the target compound according to claim 14 for providing a storage stable form of the target compound.
